# EUROPÄISCHE PATENTANMELDUNG

(11) **EP 1 905 428 A1**
(43) Veröffentlichungstag der Anmeldung: **02.04.2008**
(21) Anmeldenummer: 06121278.3
(22) Anmeldetag: 26.09.2006
(51) Int. Cl.: A61K 9/20, A61K 31/19

(54) **Ibuprofen-Brausezubereitung mit hoher Lösungsgeschwindigkeit und Verfahren zu deren Herstellung**

(71) Anmelder: Losan Pharma GmbH, 79395 Neuenburg (DE)
(72) Erfinder: Keck, Hubert, 79110, Freiburg (DE); Gruber, Peter, 79249, Merzhausen (DE)
(74) Vertreter: UEXKÜLL & STOLBERG

(57) **Zusammenfassung**

Ibuprofen-Brausezubereitungen, die (a) ein wirkstoffhaltiges Granulat, das ein wasserlösliches Ibuprofensalz und einen basischen Hilfsstoff enthält, und (b) ein Brausegranulat enthalten, das eine Säurekomponente und eine Kohlendioxid bildende Komponente enthält, und Verfahren zu deren Herstellung.

## Beschreibung

Die Erfindung betrifft Brausezubereitungen mit dem Wirkstoff Ibuprofen, die sich in Wasser schnell unter Bildung einer klaren Lösung auflösen, sowie ein vereinfachtes Verfahren zu deren Herstellung.

Ibuprofen (2-(4-Isobutylphenyl)propionsäure) ist ein bekannter und seit vielen Jahren bewährter, nicht steroidaler Wirkstoff mit analgetischer und entzündungshemmender Wirkung. Es wird unter anderem zur Behandlung von Kopfschmerzen und entzündlich bedingten Schmerzen, Schwellungen und Fieber eingesetzt.

Bei der Behandlung von Schmerzen ist ein sich besonders rasch aufbauender Blutspiegel an Ibuprofen gewünscht. Bei Ibuprofen ist bewiesen, daß ein erhöhter Plasma-Ibuprofenspiegel zu einem erhöhten analgetischen Effekt führt. Bei der Untersuchung zahlreicher Arzneiformen zeigte sich, daß gelöstes Ibuprofen zu einem früheren Beginn der Schmerzbefreiung führt als Dragees oder Filmtabletten.

Bekanntlich führen Brausetabletten, gelöst in Wasser, unter allen oralen Arzneiformen am schnellsten zum Aufbau eines hohen Blutspiegels des Wirkstoffs. Deshalb sind sie besonders dann angezeigt, wenn eine rasche und sichere Schmerzbefreiung gewünscht ist. Der gelöste Wirkstoff wird durch das relativ große Flüssigkeitsvolumen schnell über den Magen in den oberen Darmabschnitt transportiert, wo das Ibuprofen im wesentlichen resorbiert wird.

Ibuprofen ist jedoch eine organische Säure und in Wasser nahezu unlöslich. Erst im pH-Bereich ab etwa 6 geht es durch Salzbildung nach und nach in Lösung. Eine rasche und sichere Auflösung des Ibuprofens ist bei der Entwicklung einer Brausetablette besonders wichtig, weil nicht gelöste Wirkstoffpartikel beim Trinken an den Schleimhäuten haften bleiben. Infolge des neutralen bis schwach alkalischen pH-Wertes des Speichels lösen sich diese Partikel langsam auf, wobei neben einem schlechten Geschmack Brennen und lokale Irritationen an den Schleimhäuten auftreten.

Die Herstellung von löslichen Salzen des Ibuprofens und deren Einsatz in Brausetabletten oder Granulaten, die in Wasser gelöst werden, ist Gegenstand zahlreicher Patente und Patentanmeldungen.

Die DE 36 38 414 A1 offenbart Brausezusammensetzungen mit dem Wirkstoff Ibuprofen, welche die stark basischen Aminosäuren Arginin oder Lysin in einer den molaren Anteil übersteigenden Menge zur Verbesserung der Auflösung des Ibuprofens enthalten. Es wird darauf hingewiesen, daß sich die Arginin- und Lysinsalze von Ibuprofen nicht zur Herstellung von Brausezusammensetzungen eignen, da sie nicht zu einer vollständigen Auflösung des Ibuprofens führen. Die Zusammensetzungen enthalten Natriumhydrogentartrat als sauren Bestandteil. Arginin und Lysin übersteigen die Kosten für den Wirkstoff Ibuprofen deutlich und sind daher für die Verwendung als pharmazeutische Hilfsstoffe zu teuer.

EP 0 369 228 A1 offenbart eine Ibuprofen-Brausezubereitung, die ein wirkstoffhaltiges, basisches Granulat und eine Säurekomponente enthält. Zur Herstellung des basischen Granulats wird ein wasserlösliches Ibuprofensalz zusammen mit einem Trägerstoff und einem Stabilisator granuliert, das Granulat mit einer Natrium- oder Kaliumcarbonatlösung besprüht und dann getrocknet. Bevorzugte Ibuprofensalze sind das Kalium- und insbesondere das Natriumsalz. Die Herstellung des basischen Granulats ist langwierig und teuer. Aus den Ausführungsbeispielen ergibt sich, daß zur Herstellung von Ibuprofengranulat für 100.000 Tabletten insgesamt 230 kg Wasser benötigt werden, die in einem Wirbelschichtgranulator bei 100 °C wieder verdampft werden. Erschwerend kommt hinzu, daß bei Brausetabletten der Wassergehalt unter 0,5 Gew.-% liegen muß, wenn die Tabletten in Verpackungen mit einem Trokkenmittel gelagert werden, und unter 0,25 Gew.% bei der Verwendung von Blisterverpackungen.

Die Herstellung wasserlöslicher Alkalimetallsalze von Ibuprofen ist beispielsweise aus der US 4,859,704 bekannt. Hierzu wird Ibuprofen in Wasser suspendiert und beispielsweise durch den Zusatz von Natrium- oder Kaliumhydrogencarbonat neutralisiert. Bei dieser Reaktion wird unter Freisetzung von Kohlendioxid und Wasser das jeweilige Ibuprofensalz gebildet. Die Freisetzung von Kohlendioxyd ist bei der Produktion in technischem Maßstab ein großes Problem. Werden beispielsweise 400 kg Ibuprofen in der beschriebenen Weise umgesetzt, so entstehen 44.800 Liter Kohlendioxid. Dies macht eine vorsichtige und langsame Durchführung der Reaktion erforderlich.

Ein weiterer Nachteil ist die erforderliche Trocknung des Produkts. Das Natriumsalz von Ibuprofen bildet ein Dihydrat, das 13,6 Gew.-% Kristallwasser enthält und für die Herstellung einer stabilen Brausetablette nicht geeignet ist, da das Kristallwasser nicht fest genug gebunden ist. Es wird im Laufe der Zeit teilweise abgegeben und bewirkt eine Reaktion des Brausekörpers in der Brausetablette.

Somit müssen die Brausetabletten entweder in Verpackungen mit einem Trockenmittel aufbewahrt oder das Kristallwasser vor der Verarbeitung entfernt werden, was extrem zeit- und kostenaufwendig ist. Außerdem neigt Ibuprofen-Natrium zum Kleben an den Tablettierwerkzeugen und läßt sich nur schwer zu Tabletten verpressen, was seine Verarbeitung erschwert und den Einsatz großer Mengen an Hilfsstoffen erforderlich macht. Ein weiterer Nachteil ist, daß sich Ibuprofen-Natrium in Wasser nur langsam auflöst. Zudem ist Ibuprofen-Natrium etwa dreimal so teurer wie die frei Säure. Das Kaliumsalz von Ibuprofen ist extrem hygroskopisch, so daß seine Trocknung ebenfalls mit erheblichem Aufwand verbunden ist. Aufgrund seiner Hygroskopizität ist dieses Salz in kommerziellen Mengen am Weltmarkt nicht erhältlich.

Die WO 94/10994 offenbart Ibuprofen-Brausetabletten auf Basis von wasserlöslichen Ibuprofensalzen wie z.B. dem Natriumsalz, die sich dadurch auszeichnen, daß der Wirkstoff eine Kristallgröße von 180 bis 800 µm hat. Die relativ große Kristallgröße soll die Herstellung der Tabletten vereinfachen. Kristalle mit der gewünschten Größe werden durch Auflösen von Ibuprofen in einem großem Überschuß an denaturiertem Alkohol und anschließende Neutralisation mit Natriumhydroxid gewonnen. Die Verwendung von Hydroxiden führt zur Bildung von Wasser, das neben der großen Menge an Alkohol durch Trocknen entfernt werden muß. Um das sich nur langsam lösende Natriumsalz des Ibuprofens in Lösung zu bringen, ist eine große Menge an Brausekörper erforderlich. Zur Herstellung der Tabletten werden daher pro 200 mg Ibuprofen 1600 mg Natriumhydrogencarbonat eingesetzt, das Tablettengewicht beträgt insgesamt etwa 2300 mg. Die Tabletten sind damit relativ groß und daher teuer in der Herstellung. Zudem gibt das aus der Reaktion des Natriumhydrogencarbonats mit der Säure entstehende Natriumsalz und der damit verbundene pH-Wert der Brausetablettenlösung einen salzigen, unangenehmen, seifigen Geschmack.

EP 0 667 149 A1 beschreibt Ibuprofen-Brausetabletten, die Natriumcarbonat, Natriumhydrogencarbonat und Kaliumhydrogencarbonat enthalten. Zur Herstellung der Tabletten wird der Wirkstoff oder ein Salz davon mit den basischen Komponenten granuliert und dann mit einem Säuregranulat gemischt, das getrennt davon hergestellt wird. Die Mischung wird anschließend zu Tabletten geformt. Die Granulierung erfolgt mit Wasser, was eine aufwendig Trocknung erforderlich macht. Die Tabletten sollen sich in Wasser innerhalb von zwei Minuten auflösen.

Die US 6,171,617 offenbart Ibuprofen-Brausezubereitungen, die zwei getrennte Granulate enthalten, (a) ein ibuprofenhaltiges Granulat und (b) ein Brausegranulat, das eine Säurekomponente und eine Kohlendioxid bildende Komponente enthält. Zur Herstellung des Wirkstoffgranulats wird Ibuprofen mit einem basischen Hilfsstoff gemischt und anschließend mit Wasser oder einer Wasser-Alkohol-Mischung granuliert. Als bevorzugte basische Hilfsstoffe werden Natriumcarbonat, Kaliumcarbonat, Natriumhydrogencarbonat, Kaliumhydrogencarbonat und Trinatriumphosphat genannt. Das Wasser wird nach der Herstellung des Granulats durch Trocknen wieder entfernt. Die beiden Granulate werden gemischt und in Beutel gefüllt oder zu Tabletten verpreßt. Die Tabletten haben ein Gewicht von 3,3 g bei einer Wirkstoffmenge von 200 mg. Das Wirkstoffhaltige Granulat (a) enthält Ibuprofen in Form seiner Säure und nicht als wasserlösliches Salz. Hierdurch soll eine zeitlich versetzte Auflösung der Granulate erzielt werden. Zunächst geht das Brausegranulat (b) in Lösung und erst dann der Wirkstoff selbst.

Weiterhin sind Brausetabletten bekannt, die Ibuprofen-Lysinat oder Ibuprofen-Arginat als Wirkstoffe enthalten. Diese Salze sind teuer und zur Herstellung von Brausetabletten wenig geeignet, da sie nicht zu einer vollständigen Auflösung des Ibuprofens führen (DE 36 38 414)).

Aus den obigen Ausführungen ergibt sich, daß die Herstellung von Ibuprofen-Brausezubereitungen aufwendig und teuer ist. Daher sind, obwohl der Wirkstoff Ibuprofen gegenüber vergleichbaren Wirkstoffen wie Acetylsalicylsäure und Paracetamol deutliche Vorteile besitzt, keine preisgünstigen und gut schmeckende Brausetablette am Markt. Eine Brausetablette mit 400 mg Ibuprofen war kurzzeitig erhältlich, fand jedoch aufgrund ihres unagenehmen Geschmacks keine Akzeptanz, eine 600 mg Brausetablette wurde bisher überhaupt noch nicht angeboten.

Der Erfindung liegt die Aufgabe zugrunde, Ibuprofen-Brausezubereitungen zur Verfügung zu stellen, die sich in Wasser unter Bildung einer klaren Lösung rasch auflösen. Die Lösung soll weiterhin einen angenehmen Geschmack aufweisen und sich wirtschaftlich herstellen lassen. Eine weitere Aufgabe der Erfindung ist die Bereitstellung eines Verfahrens, daß die schnelle und kostengünstige Herstellung solcher Tabletten erlaubt. Das Verfahren soll insbesondere keine aufwendigen Trocknungsschritte erfordern.

Diese Aufgabe wird erfindungsgemäß durch Ibuprofen-Brausezubereitungen gelöst, die
(a) ein wirkstoffhaltiges Granulat, das Ibuprofen und einen basischen Hilfsstoff enthält, und
(b) ein Brausegranulat enthalten, das eine Säurekomponente und eine Kohlendioxid bildende Komponente enthält.

Die Zubereitungen sind dadurch gekennzeichnet, daß das wirkstoffhaltige Granulat das Ibuprofen in Form eines wasserlöslichen Salzes, vorzugsweise das Kaliumsalz von Ibuprofen oder eine Mischung des Kalium- und des Natriumsalzes enthält. Zubereitungen, die den Wirkstoff Ibuprofen ausschließlich in Form des Kaliumsalzes oder einer Mischung des Kalium- und Natriumsalzes enthalten, sind besonders bevorzugt. Das Kaliumsalz und das Natriumsalz werden vorzugsweise in einem molaren Verhältnis von Kalium- zu Natriumsalz von 1:0 bis 1:1, besonders bevorzugt 1:0,02 bis 1: 0,1 ganz besonders bevorzugt 1:0,05 eingesetzt.

Ibuprofen existiert in zwei stereoisomeren Formen, der R(-)-Form und der S(+)-Form. Die R(-)-Form ist wesentlich weniger pharmakologisch aktiv als die S(+)-Form. Wenn nicht anders angegeben wird unter Ibuprofen oder Ibuprofensalz das jeweilige Racemat verstanden. Alle Mengenangaben beziehen sich auf das Racemat, wobei im Fall von Salzen, wenn nicht anders angegeben, die korrespondierende Menge an freier Säure aufgeführt wird. Neben dem Racemat ist die Verwendung der S(+)-Form bevorzugt. Diese wird in der halben Menge wie das Racemat eingesetzt. Unter Ibuprofen wird hierin, wenn nicht anders angegeben, die freie Ibuprofen-Säure verstanden.

Als basische Komponente enthält das wirkstoffhaltige Granulat vorzugsweise eine Base (Base 2), die aus einer ersten Base (Base 1) bei der Reaktion mit Ibuprofen entsteht. Bei dieser Reaktion geht Ibuprofen in das entsprechende Ibuprofensalz über, und die Base 1 bildet unter Aufnahme eines Protons die Base 2.

Im Fall der als Base 1 bevorzugten, nachstehend beschriebenen basischen Stoffe entsteht als Base 2 NaHCO₃, KHCO₃, Dikaliumcitrat, Dinatriumcitrat, Dinatriumphosphat, Dikaliumphosphat, die protonierten Formen von Lysin, Arginin, physiologisch verträglichen, basischen organischen Aminen, wie Meglumin (N-Methylglucamin).

Da die Base 1 vorzugsweise im Überschuß eingesetzt wird, kann die basische Komponente auch Mischungen von Base 1 und Base 2 und gegebenenfalls auch Mischungen unterschiedlicher Basen 1 und/oder Basen 2 enthalten. Vorzugsweise enthält das wirkstoffhaltige Granulat als basische Komponente KHCO₃, eine Mischung von KHCO₃ und K₂CO₃, eine Mischung von KHCO₃ und NaHCO₃ oder eine Mischung von KHCO₃, K₂CO₃, NaHCO₃ und Na₂CO₃.

Das Verhältnis von Ibuprofensalz zu basischem Hilfsstoff in dem wirkstoffhaltigen Granulat liegt vorzugsweise im Bereich von 0,8 bis 2 mol, besonders bevorzugt 1 bis 1,5 mol und ganz besonders bevorzugt 1,1 bis 1,25 mol basischem Hilfsstoff pro mol Ibuprofensalz.

Neben dem wasserlöslichen Ibuprofensalz und dem basischen Hilfsstoff kann das wirkstoffhaltige Granulat weitere Hilfsstoffe enthalten, beispielsweise Stoffe zur Verbesserung der Löslichkeit, Benetzbarkeit, Verpreßbarkeit und des Fließverhaltens. Bevorzugte weitere Hilfsstoffe sind Bindemittel, wie Povidone und Celluloseester, neutrale wasserlösliche Hilfsstoffe, wie Sorbit, Mannit, Maltit, Isomaltit, schwach basische Hilfsstoffe. Insgesamt wird jedoch angestrebt, neben den weiter oben genannten basischen Hilfsstoffen keine oder nur geringe Mengen an weiteren Hilfsstoffen einzusetzen. Granulate, die neben dem Ibuprofensalz und den basischen Hilfsstoffen keine weiteren Hilfsstoffe enthalten, sind daher besonders bevorzugt.

Die Brausezubereitung enthält als zweite Komponente ein Brausegranulat, das als Säurekomponente vorzugsweise eine physiologisch unbedenkliche, anorganische oder vorzugsweise organische Genußsäure oder ein saures Salz davon, insbesondere Zitronensäure, Weinsäure, Apfelsäure, Ascorbinsäure, ein saures Salz dieser Säuren, wie Mononatriumcitrat, Mononatriumtartrat oder Mononatriumfumarat, ein saures anorganisches Salz, wie Monokaliumphosphat, Kaliumhydrogensulfat oder Natriumhydrogensulfat, Glutaminsäure, Adipinsäure, Glutaminsäure-Hydrochlorid, Betainhydrochlorid oder eine Mischung davon enthält.

Bevorzugte Kohlendioxid bildende Komponenten sind Alkali-oder Erdalkalicarbonate oder -hydrogencarbonate, insbesondere NaHCO₃, NaCO₃, KHCO₃, K₂CO₃, Natriumglycincarbonat und Mischungen davon, ganz besonders bevorzugt NaHCO₃, KHCO₃ oder eine Mischung davon.

Neben der Säurekomponente und der Kohlendioxid abspaltenden Komponente enthält das Brausegranulat üblicherweise weitere Hilfsstoffe, beispielsweise Bindemittel, wie Povidone oder Methylhydroxypropylcellulosen, um gut strukturierte nicht staubförmige Brausekörper zu gewährleisten. Die Brausekörper können auch neutrale, gut wasserlösliche Hilfsstoffe wie Saccharose, Glucose, Sorbit, Mannit, Xylit und Maltit enthalten, die zur Steuerung der Reaktivität des Brausekörpers dienen.

Die erfindungsgemäßen Brausezubereitungen enthalten zur besseren Benetzung der Bestandteile der Zubereitung und zur Beschleunigung des Zerfalls vorzugsweise mindestens ein physiologisch verträglichen Tensid. Besonders geeignet sind Tenside mit einem HLB-Wert > 12, vorzugsweise 12 bis 18, besonders bevorzugt 14 bis 16, wie z. B. Natriumlaurylsulfat oder Saccharosepalmitat. Das oder die Tenside werden vorzugsweise in einer Menge von 0,01 bis 0,1 Gewichtsteilen, bevorzugt 0,025 bis 0,035 Gewichtsteilen pro Gewichtsteil Ibuprofen eingesetzt. Die Tenside können sowohl im Wirkstoffgranulat als auch im Brausegranulat enthalten sein, bevorzugt werden sie dem Brausegranulat zugegeben.

Weiterhin können die Zubereitungen zur Verbesserung des Geschmackes Süßmittel und wasserlösliche oder in Wasser dispergierbare Aromen enthalten. Als Süßmittel sind neben Zuckern wie Saccharose insbesondere Süßstoffe bevorzugt, da sie über eine hohe Süßkraft verfügen und damit zur Herstellung kleiner, preiswerter Brausetabletten besonders geeignet sind. Bevorzugte Süßstoffe sind Aspartam, Natriumsaccharinat, Natriumcyclamat, Acesulfam K, Neohesperidin, Sucralose und Gemische der genannten Süßstoffe. Süßstoffe werden bevorzugt in einer Menge von 5 bis 100 mg, bevorzugt 10 bis 50 mg und ganz bevorzugt 20 bis 30 mg pro 200 mg Ibuprofen eingesetzt.

Bevorzugte Aromen sind Pfefferminz-, Menthol- und Vanillearoma, besonders Zitrusaromen wie z.B. Grapefruitaroma. Aromen werden bevorzugt in einer Menge von weniger als 100 mg, bevorzugt weniger als 75 mg pro 200 mg Ibuprofen eingesetzt. Aromen werden bevorzugt in einer Menge von 10 bis 100 mg, bevorzugt 15 bis 75 mg und ganz besonders bevorzugt 25 bis 40 mg pro 200 mg Ibuprofen eingesetzt.

Das wirkstoffhaltige Granulat und/oder das Brausegranulat können neben Ibuprofen noch weitere Wirkstoffe enthalten, beispielsweise Antihistamine, schleimhautabschwellende Wirkstoff, Antacida, Analgetika, wie Aspirin oder Paracetamol, Expektorantien, anästhesierende Wirkstoffe und Kombinationen davon. Besonders bevorzugte Wirkstoffe sind Diphenhydramin, Chlorpheniraminmaleat, Brompheniraminmaleat, Phenylpropanolamin, Phenylephrinhydrochlorid, Pseudoephedrinhydrochlorid, Codein, Ascorbinsäure und Coffein.

Die Brausezubereitung liegen vorzugsweise in der Form einer Dosierungseinheit vor, besonders bevorzugt in Form von Brausetabletten oder von in Sachets oder Stickpacks abgefülltem Trinkgranulat, d.h. einem Ibuprofen-Brausegranulat, das das Wirkstoffgranulat (a) und Brausegranulat (b) z.B. in Form einer Mischung enthält.

Das wirkstoffhaltige Granulat (a) zeichnet sich durch eine hohe Löslichkeit und insbesondere Lösungsgeschwindigkeit aus, so daß das wirkstoffhaltige Granulat und das Brausegranulat praktisch zeitgleich in Lösung gehen. Die Auflösezeit ist gegenüber Granulaten mit zeitlich versetzter Auflösung deutlich reduziert. Die Lösungsgeschwindigkeit des wirkstoffhaltigen Granulats beträgt vorzugsweise 5 bis 20 Sekunden, besonders bevorzugt 5 bis 15 Sekunden und ganz besonders bevorzugt 5 bis 12 Sekunden, so daß das Granulat kaum den Boden des Meßgefäßes erreicht. Die Partikelgröße des Granulats liegt vorzugsweise im Bereich von 0,1 bis 1,25 mm, besonders bevorzugt 0,1 bis 0,9 mm.

Durch diese bevorzugten Eigenschaften kann die Menge an notwendigem Brausekörper zur Herstellung einer sich in 2 bis 3 Minuten auflösenden Brausetablette wesentlich reduziert werden. Es lassen sich beispielsweise Brausetabletten herstellen, die bei einer Dosierung von 200 mg Ibuprofen weniger als 1,5 g wiegen, sich aber trotzdem klar in Wasser auflösen und durch die geringe Menge an Brausekörper und insbesondere Natriumhydrogencarbonat einen angenehmen Geschmack aufweisen. Damit werden auch Brausetabletten zugänglich, die selbst bei einer Wirkstoffmenge von 600 mg Ibuprofen einen annehmbaren Geschmack haben.

Die Lösungsgeschwindigkeit von Granulaten wird mit einer Dissolution-Apparatur gemäß der europäischen Pharmacopoe gemessen. Hierzu wird die so genannte Paddle-Methode verwendet, bei der in ein Gefäß 500 ml entmineralisiertes Wasser mit einer Temperatur von 20 °C eingefüllt werden. Der Gefäßinhalt wird mit einer Umdrehungsgeschwindigkeit 100 Umdrehungen pro Minute gerührt, und 1,0 g des zu messenden Granulats werden zugegeben.

Brausetabletten müssen sich entsprechend ihrer Monographie in allen Pharmacopoen praktisch selbständig ohne Rühren auflösen. Somit kommt der Brausekomponente auch die Aufgabe zu, sicherzustellen, daß eventuell gebildeter Bodensatz durch eine ausreichend starke CO₂-Bildung aufgelöst wird.

Die erfindungsgemäßen Zubereitung enthalten vorzugsweise 200 bis 800 mg Ibuprofen (gemessen als freie Säure) pro Dosierungseinheit. Brausetabletten enthalten vorzugsweise 200, 400 oder 600 mg Ibuprofen, Sachets bzw. Stickpacks 200, 400, 600 oder 800 mg Ibuprofen pro Dosierungseinheit.

Die Dosierungseinheiten haben im Falle von Brausetabletten vorzugsweise ein Gesamtgewicht von 1000 bis 1500 mg pro 200 mg Ibuprofen (gemessen als freie Säure). Bevorzugte Gesamtgewichte für eine Dosierungseinheit sind demnach:
- 200 mg Ibuprofen:: 1000 bis 1500 mg, bevorzugt 1300 bis 1400 mg;
- 400 mg Ibuprofen:: 2000 bis 3000 mg, bevorzugt 2400 bis 2800 mg;
- 600 mg Ibuprofen:: 3000 bis 4500 mg, bevorzugt 3500 bis 3900 mg.

Die erfindungsgemäßen Zubereitungen enthalten weiterhin vorzugsweise insgesamt 3 bis 5 Gewichtsteile, besonders bevorzugt 3,5 bis 4,2 Gewichtsteile basische Komponenten wie Alkalihydrogencarbonat und insbesondere Natriumhydrogencarbonat pro Gewichtsteil Ibuprofen.

Weiter enthalten die erfindungsgemäßen Zubereitungen pro Gewichtsteil Ibuprofen vorzugsweise 1 bis 2 Gewichtsteile, besonders bevorzugt 1,4 bis 1,8 Gewichtsteile saure Komponenten wie Citronensäure und/oder Mononatriumcitrat.

Brausegranulat (b) und Wirkstoffgranulat (a) werden im Fall von Brausetabletten bevorzugt in einem Verhältnis von 1,7 bis 2,9 Gewichtsteilen Brausegranulat (b), besonders bevorzugt 2,1 bis 2,6 Gewichtsteilen Brausegranulat (b) pro 1 Gewichtsteil Wirkstoffgranulat (a) eingesetzt.

Dabei enthalten die Zubereitungen die angegebenen Komponenten in solchen Mengen, daß die durch Auflösung einer Dosierungseinheit in 200 ml Wasser erhaltene trinkfertigen Lösung einen pH-Wert von 6,3 bis 7,5, vorzugsweise < 7, insbesondere 6,5 bis 6,8 und ganz besonders bevorzugt 6,6 bis 6,7 aufweist, so daß die Lösung durch physikalisch in Wasser gelöstes Kohlendioxid auf der Zunge prickelnd ist und weder einen salzigen noch einen seifigen Geschmack aufweist.

Die erfindungsgemäßen Zubereitungen unterscheiden sich damit nicht nur durch ihr besonders geringes Gewicht pro Dosierungseinheit sondern auch durch einen wesentlich besseren, nicht salzigen oder seifigen Geschmack von bekannten Brausezubereitungen. Außerdem ergeben die Zubereitungen nach dem Auflösen vollkommen klare Lösungen.

Bei Trinkgranulaten, die in Sachets oder in Stickpacks abgefüllt werden, kann die Menge an Brausegranulat (b) gegenüber dem Wirkstoffgranulat (a) noch weiter reduziert werden, so daß die genannten Vorteile besonders ausgeprägt sind. Trinkgranulate haben pro Dosierungseinheit vorzugsweise ein Gesamtgewicht von 500 bis 950 mg pro 200 mg Ibuprofen (gemessen als freie Säure). Bevorzugte Gesamtgewichte für eine Dosierungseinheit Ibuprofen-Trinkgranulat sind demnach:
- 200 mg Ibuprofen:: 500 bis 950 mg, bevorzugt 550 bis 800 mg;
- 400 mg Ibuprofen:: 1000 bis 1900 mg, bevorzugt 1100 bis 1600 mg;
- 600 mg Ibuprofen:: 1500 bis 2850 mg, bevorzugt 1700 bis 2400 mg;
- 800 mg Ibuprofen:: 2000 bis 3800 mg, bevorzugt 2300 bis 3200 mg.

Das Mengenverhältnis von Wirkstoffgranulat (a) zu Brausegranulat (b) liegt bei Trinkgranulaten vorzugsweise im Bereich von 0,4 bis 1,6 Gewichtsteilen Brausegranulat (b), bevorzugt 0,6 bis 1,25 Gewichtsteilen Brausegranulat (b) pro Gewichtsteil Wirkstoffgranulat (a).

Der Erfindung liegt weiterhin die Aufgabe zugrunde, ein wirtschaftliches Verfahren zur Herstellung von Ibuprofen-Brausezubereitungen zur Verfügung zu stellen. Ein wesentlicher Kern der Lösung dieser Aufgabe ist ein Verfahren, daß die schnelle und wirtschaftliche Umwandlung des unlöslichen Ibuprofens in ein hoch wasserlösliches Granulat selbst unter Produktionsbedingungen ermöglicht.

Erfindungsgemäß wird das wirkstoffhaltige Granulat (a) vorzugsweise herstellt, indem man Ibuprofen in Form seiner freien Säure mit einem oder mehreren basischen Hilfsstoffen (Base 1) ohne Zugabe von Wasser intensiv mischt.

Vorzugsweise mischt man das Ibuprofen und den basischen Hilfsstoff unter Zugabe eines organischen Lösungsmittels miteinander. Besonders geeignete organische Lösungsmittel sind Isopropanol, Äthanol, Aceton und Mischungen davon, ganz besonders Isopropanol. Das organische Lösungsmittel wird vorzugsweise in einer Menge von 0 bis 10 Gew.-%, besonders bevorzugt 0,5 bis 3 Gew.-% und ganz besonders bevorzugt 0,8 bis 1,5 Gew.-% bezogen auf das Gesamtgesicht der Reaktionsmischung. eingesetzt. Zur Beschleunigung der Reaktion kann die Mischung erwärmt werden, vorzugsweise auf eine Temperatur im Bereich von 50 bis 60 °C.

Es wurde völlig überraschend gefunden, daß sich das Ibuprofen beim intensiven Mischen mit den basischen Hilfsstoffen ohne die Zugabe von Wasser vollständig umsetzt. Nach Zusatz einer sehr kleinen Menge eines organischen Lösungsmittels erwärmt sich das Gemisch durch eigene Reaktionswärme auf ca. 40 bis 55 °C unter vollständiger Umwandlung in ein hochwasserlösliches Ibuprofen-Salzgemisch.

Werden beispielsweise in einem geeigneten Mischbehälter 360 kg Ibuprofen, 270 kg Kaliumcarbonat, 9 kg Natriumcarbonat und 6 kg Isopropanol (0,9 %) bei Raumtemperatur gemischt, steigt innerhalb von etwa 30 Minuten die Produkt-temperatur auf 40 bis 50 °C an. Das Produkt ist während des gesamten Prozesses zunächst pulverförmig und geht dann in ein sehr feines Granulat über, ohne daß die Mischung klebrig wird oder ihre Fließfähigkeit verliert. Erstaunlicherweise läuft die Reaktion in festem Zustand ab. Entnimmt man nach etwa 40 Minuten eine Probe aus der gerührten Mischung, so löst sich diese in Wasser innerhalb von wenigen Sekunden quantitativ auf und ergibt eine völlig klare Lösung.

Benutzt man für diesen Prozeß einen Vakuumgranulator, so kann man infolge der Produktwärme und des Vakuums das eingesetzte Isopropanol innerhalb von weniger als 5 Minuten weitgehendst entfernen. Somit ist der Gesamtprozeß zur Umsetzung von 360 kg Ibuprofen (ausreichend für 1,8 Millionen Brausetabletten mit 200 mg Ibuprofen) in weniger als 45 Minuten beendet. Selbst 600 kg Ibuprofen können in weniger als 60 Minuten umgesetzt und getrocknet werden. Die Geschwindigkeit der Reaktion und insbesondere die Eindeutigkeit des Reaktionsverlaufes waren nicht vorhersehbar und sind völlig unerwartet.

Als basische Hilfsstoffe (Base 1) eignen sich wasserlösliche basische Substanzen, die in 0,1 molarer wäßriger Lösung einen pH-Wert > 11,0 aufweisen. Bevorzugte basische Hilfsstoffe (Base 1) sind Trikaliumphosphat, Trinatriumphosphat, Trikaliumcitrat, Trinatriumacitrat sowie die entsprechenden Natriumsalze, das Kalium- und das Natriumsalz des Glycins, Lysin, Arginin und physiologisch unbedenkliche organische Amine, wie Meglumin (N-Methylglucamin), insbesondere Alkalicarbonate wie Natriumcarbonat und Kaliumcarbonat sowie Mischungen dieser Stoffe.

Die basischen Hilfsstoffe werden vorzugsweise in einer Menge von 0,8 bis 2 mol, bevorzugt 1,0 bis 1,5 mol und ganz bevorzugt 1,1 bis 1,25 mol basischem Hilfsstoff pro mol Ibuprofen verwendet, wobei kaliumhaltige basische Hilfsstoffe und besonders Mischungen von natriumhaltigen und kaliumhaltigen basischen Hilfsstoffen bevorzugt sind. Kalium- und Natriumsalze, wie beispielsweise K₂CO₃ und Na₂CO₃, werden vorzugsweise in einem molaren Verhältnis (K : Na) von 1 : 0 bis 1 : 1, vorzugsweise 1 : 0,02 bis 1 : 0,1, besonders bevorzugt 1 : 0,05 einsetzt.

Besonders bevorzugte basische Hilfsstoffe sind Kaliumcarbonat und Mischungen von Kaliumcarbonat und Natriumcarbonat. Neben diesen basischen Hilfsstoffen können noch weitere basische Hilfsstoffe zugemischt werden, die vorzugsweise aus der Gruppe der oben genannten basischen Hilfsstoffe (Base 1) ausgewählt sind. Weitere basische Hilfsstoffe werden vorzugsweise in einer Menge von 0 bis 0,5 mol der genannten Gesamtmenge an basischen Hilfsstoffen von 0,8 bis 2,0 mol pro mol Ibuprofen eingesetzt.

Die mittlere Partikelgröße des Ibuprofens und der alkalischen Hilfsstoffe sollte nicht größer als 0,18 mm, bevorzugt nicht größer als 0,125 mm und ganz besonders bevorzugt nicht größer als 0,1 mm sein. Mindestens 95 % aller an der Reaktion beteiligten Komponenten sollten kleiner als 0,25 mm sein. Aus Kostengründen sind in allen Fällen Partikel mit einer minimalen mittleren Partikelgröße von 0,025 mm und besonders 0,05 mm bevorzugt. Die mittlere Partikelgröße wird gemäß DIN 66145 ermittelt, d.h. aus der dem Fachmann bekannten Rosin-Rammler-Sperling-Bennet-(RRSB-) Kurve entnommen.

Das Ibuprofen reagiert mit der oder den basischen Verbindungen (Base 1) unter Bildung eines innigen Salzgemisches, das Ibuprofensalz und das Reaktionsprodukt (Base 2) der basischen Verbindung 1 und ggf. Überschüsse der Base 1 enthält. Bei der Verwendung von Kaliumcarbonat als basischer Verbindung 1 entsteht Ibuprofen-Kalium und Kaliumhydrogencarbonat als Base 2. Es ist völlig unerwartet, daß unter den geschilderten milden Reaktionsbedingungen die Kristalle des Ibuprofens und des basischen Hilfsstoffs bis in den molekularen Zustand unter Salzbildung total durchreagieren, ohne das dabei sichtbar eine Lösung oder eine Schmelze auftritt. Es entsteht eine extrem innige, sehr homogene Mischung zwischen dem Ibuprofensalz und dem basischen Hilfsstoff, wobei Mischungen, die Ibuprofen-Kalium und Kaliumhydrogencarbonat enthalten, ganz besonders bevorzugt sind. Sowohl das Kaliumsalz von Ibuprofen als auch das gebildete Kaliumhydrogencarbonat sind sehr gut wasserlöslich und tragen zu der hohen Löslichkeit und der extrem hohen Lösungsgeschwindigkeit des erfindungsgemäßen wirkstoffhaltigen Granulats bei. Das wirkstoffhaltige Granulat kann zudem kleine Hohlräume aufweisen, die vermutlich durch den Eischluß von CO₂ entstehen. Diese Hohlräume weisen beispielsweise Durchmesser von 10 bis 40 µm, insbesondere ca. 30 µm auf und können dem Granulat eine bienenwabenähnliche Struktur verleihen.

Weiterhin wurde gefunden, daß das mit dem Ibuprofen-Salz innig verbundene Hydrogencarbonat bei der Herstellung oder Auflösung der Brausezubereitung die Rückreaktion des Wirkstoffsalzes mit der im Brausegranulat enthaltenen Säure zu unlöslichem Ibuprofen nahezu vollständig unterdrückt. Dies ist ein entscheidender Grund für die klare Auflösung der erfindungsgemäßen Ibuprofen-Brausezubereitungen und für den erstaunlich niedrigen pH-Wert der entstehenden Brauselösung.

Gemäß einer bevorzugten Ausführungsform des erfindungsgemäßen Verfahrens wird als basischer Hilfsstoff eine Mischung von Kalium- und Natriumsalzen, insbesondere eine Mischung von Kaliumcarbonat und Natriumcarbonat verwendet. Obwohl das durch den Zusatz von Natriumcarbonat entstehende Ibuprofen-Natrium weniger gut löslich ist als das Kaliumsalz, wird ein sehr gut wasserlösliches Ibuprofen-Salzgemisch erhalten. Die Zugabe einer geringen Menge einer Natriumverbindung hat den Vorteil, daß das entstehende Ibuprofen-Natrium aufgrund seiner Klebrigkeit als Bindemittel wirkt und die Verarbeitbarkeit des Wirkstoffgranulats verbessert.

Außerdem entsteht in Abwesenheit von Wasser wasserfreies Ibuprofen-Natrium, daß aufgrund seiner Tendenz zur Bildung eines Dihydrats zur Aufnahme von Wasser neigt und beispielsweise durch die Ausgangsstoffe eingeschlepptes oder in geringen Mengen gebildetes Wasser unter Bildung von Ibuprofen-Natrium-Dihydrat bindet. Es fungiert somit als inneres Trockenmittel. Unter den zur Herstellung von Brausetabletten üblichen Produktionsbedingungen von ca. 20 bis 25 °C und ca. 20 % relativer Feuchtigkeit ist das Granulat ausreichend stabil und nimmt nur unbedeutende Mengen an Wasser aus der Luft auf. Das im Vakuumgranulator hergestellte Granulat kann ohne Probleme gesiebt und gemischt werden.

Die erfindungsgemäß hergestellten Ibuprofen-Salzgemische zeichnet sich neben ihrer hohen Lösungsgeschwindigkeit auch durch eine bessere Verpreßbarkeit und eine geringe Neigung zum Kleben aus.

Der Wassergehalt des Granulats soll möglichst im Bereich von < 0,5 Gew.-%, besonders bevorzugt < 0,25 Gew.-% liegen. Im Anschluß an die Reaktion wird das Granulat daher erforderlichenfalls getrocknet. Ein Wassergehalt von < 0,5 Gew.-% und insbesondere 0,25 Gew.-% ist notwendig, um eine ausreichende Lagerstabilität der Brausezubereitungen zu gewährleisten. Diese müssen über Jahre stabil sein und dürfen bei kurzzeitigen Temperaturstreß keine Kohlendioxidentwicklung durch Reaktion mit den Brausegranulat zeigen.

Granulate mit einem Wassergehalt mit < 0,5 Gew.-% eignen sich besonders für Brausezubereitungen, die in Verpackungen mit einem Trockenmittel verpackt werden, beispielsweise in Röhren mit einem Trockenstopfen. Bei der Verwendung von z.B. Blisterverpackungen oder Alu/Alu-Folien sollte der Wassergehalt unter 0,25 Gew.-% liegen.

Wesentlich für den Reaktionsverlauf ist die Vermeidung des Zusatzes von Wasser. Es werden daher vorzugsweise keine basischen Hilfsstoffe eingesetzt, die bei der Reaktion mit der Ibuprofensäure Wasser bilden, wie z.B. KOH und NaOH oder auch Kaliumhydrogencarbonat und Natriumhydrogencarbonat. Bicarbonate haben den zusätzlichen Nachteil, daß bei der Reaktion mit Säure neben Wasser auch Kohlendioxid entsteht.

Die Trocknung wasserhaltiger Granulate ist energieintensiv und aufwendig. Zur Erzielung eines Wassergehalts von weniger als 0,5 Gew.-% sind unter Produktionsbedingungen bei einem 500 kg Ansatz selbst im Vakuum mehr als 20 Stunden erforderlich, sofern bei der Herstellung des wirkstoffhaltigen Granulats Wasser eingesetzt wird. Ein weiteres, erst unter Produktionsbedingung in 500 kg Maßstab erkanntes Problem liegt darin, daß bei Anwesenheit von Wasser die Reaktion von Ibuprofen mit Carbonaten nicht einheitlich abläuft. Das Ibuprofen reagiert zwar bevorzugt mit dem basischeren Carbonat unter Bildung von Hydrogencarbonat, in Gegenwart von Wasser findet jedoch auch eine Reaktion mit dem schwächer basischen Hydrogencarbonat unter Bildung von Kohlendioxid und Wasser nach folgendem Reaktionsschema statt:
Ibuprofen + K₂CO₃ → Ibuprofen-Kalium + KHCO₃
KHCO₃ + Ibuprofen → Ibuprofen-Kalium + CO₂ + H₂O

Das Ausmaß dieser Reaktionen ist von der Mischungsintensität, der Temperatur des Produktes und anderen Parametern abhängig und damit unter Produktionsbedingungen nur schwer kontrollierbar, so daß nicht reproduzierbare Massenverluste eintreten. Bei jedem Batch muß daher die Zusammensetzung analysiert und entsprechend dem gefunden Gehalt an Wirkstoff neu justiert werden. Die Trocknung wird zusätzlich dadurch erschwert, daß sich Natriumhydrogencarbonat bereits ab einer Temperatur von etwa 60 °C unter Bildung von Wasser und Kohlendioxid zu zersetzten beginnt, wodurch die obigen Probleme noch verschärft werden. Allein verantwortlich für dieses Verhalten ist das vorhandene Wasser, das die eingesetzten Carbonate und Hydrogencarbonate löst, die dann mit dem in Wasser unlöslichen Ibuprofen reagieren.

Erfindungsgemäß wurde demgegenüber gefunden, daß die Reaktion in Abwesenheit von Wasser selbst im 600 kg-Maßstab stets reproduzierbar verläuft und ein wirkstoffhaltiges Granulat mit einem Wirkstoffgehalt zwischen 100 und 101 % ergibt (bezogen auf den theoretischen Wert). Diese kleine Abweichung wird vermutlich durch die Reaktion geringer Mengen von während des Prozesses gebildeten Hydrogencarbonaten zu CO₂ und Wasser und/oder eine geringfügige Aufkonzentration des Granulates durch Trocknung der ungetrocknet eingesetzten Komponenten verursacht. Da praktisch keine Erhöhung der Konzentration des Ibuprofens stattfindet, ist zu schließen, daß zur Überraschung des Fachmanns praktisch nur Carbonat mit dem Ibuprofen reagiert und ein Proton unter Bildung von Hydrogencarbonat aufnimmt. Die bei Zugabe von Wasser unter Produktionsbedingungen beobachtete Reaktion von Hydrogencarbonaten mit Ibuprofen findet nicht statt, und dadurch bedingte schwankende, nicht reproduzierbare Wirkstoffgehalte treten nicht auf. Da die Umsetzung und Trocknung vorzugsweise bei Temperaturen unterhalb von 60 °C ablaufen, ist auch die thermische Zersetzung von gebildetem Natriumhydrogencarbonat in CO₂ und Wasser ausgeschlossen.

Das erfindungsgemäße Verfahren kann in jedem geeignetem Mischbehälter durchgeführt werden. Vorzugsweise wird das Mischen in einem Vakuumgranulator, Kompaktor, Wirbelschichtreaktor oder Extruder durchführt.

Bei der Verwendung von organischen Lösungsmitteln ist der Einsatz eines Vakuumgranulators besonders vorteilhaft, insbesondere eine Vakuumgranulators, der neben den Einrichtungen zur Mischung der Komponenten auch die Möglichkeit der sofortigen Trocknung vorsieht. In einem Vakuumgranulator lassen sich die geringen Mengen an Lösungsmitteln, die erfindungsgemäß eingesetzt werden, in wenigen Minuten entfernen.

Zur reinen thermischen Solubilisierung des Ibuprofens ohne Lösungsmittel werden vorzugsweise Kompaktoren, Wirbelschichtreaktoren oder Extruder eingesetzt. Hierbei wird das im wesentlichen aus Ibuprofen und basischen Hilfsstoffen bestehende Gemisch unter Rühren auf ca. 50 °C erwärmt. Unter diesen Bedingungen läßt sich eine Batch-Größe von ca. 500 kg innerhalb von 2 Stunden in ein gut wasserlösliches Wirkstoffgranulat überführen.

Bei der lösungsmittelfreien Umsetzung in einem Kompaktor wird das Gemisch aus Ibuprofen und den genannten alkalischen Hilfsstoffen zwischen zwei Walzen stark verdichtet. Die dabei entstehende Reaktionswärme reicht zur Durchführung der Reaktion aus, zusätzliches Erwärmen ist nicht erforderlich.

Überraschenderweise kann die thermische Umsetzung des Ibuprofens mit den genannten alkalischen Hilfsstoffen auch in einer Wirbelschicht erfolgen. Dabei wird das Gemisch durch die Prozeßluft auf 50 auf 70 °C erwärmt.

Gemäß einer weiteren bevorzugten Variante kann die lösungsmittelfreie Umsetzung in einen Extruder durchgeführt werden. Hierbei werden Ibuprofen und alkalische Hilfsstoffe mittels gravimetrisch arbeitender Dosierwaagen genau in einen Extruder eindosiert und miteinander gemischt. Die Extrusion wird bevorzugt unter den im folgenden beschriebenen Bedingungen durchgeführt. Die Segmente des Extruders, in denen die Durchmischung erfolgt, werden dabei auf 60 bis 100 °C erwärmt. Die anschließenden Segmente des Extruders sind gekühlt, so daß die Masse den Extruder vorteilhaft als ein körniges, staubarmes Granulat mit einer Temperatur von ca. 40 °C verläßt. Infolge der höheren Temperatur kann hier ggf. in geringem Umfang eine thermische Zersetzung von Natriumhydrogencarbonat und/oder eine Reaktion von Hydrogencarbonat mit Ibuprofen stattfinden. Das extrudierte Material weist in der Regel einen Wirkstoffgehalt von 101,5 bis 102,5 % auf. Sobald die Produkttemperatur ca. 65 °C übersteigt muß mit einer geringfügigen Reaktion von Hydrogencarbonaten mit noch nicht umgesetztem Ibuprofen und insbesondere im Falle von Natriumhydrogencarbonat mit einer thermischen Zersetzung gerechnet werden. Trotz der höheren Reaktionstemperatur findet diese Nebenreaktion nur in einem erstaunlich geringen Umfang statt. Der Wassergehalt des extrudierten Materials liegt gewöhnlich in einer Größenordnung von 0,5 Gew.-%.

Durch Extrusion können Granulate hergestellt werden, die kleine Hohlräume aufweisen und die eine bienenwabenähnliche Struktur haben. Derartige Granulate eignen sich besonders zur Herstellung von Trinkgranulaten.

Das wirkstoffhaltige Granulat wird anschließend mit einem Brausegranulat gemischt und dann vorzugsweise in Sachets oder Stickpacks verpackt oder zu Tabletten verpreßt.

Das Brausegranulat (b) wird in einem in einem getrennten Arbeitsgang hergestellt, vorzugsweise in einem Vakuumgranulator. Dabei wird eine Säurekomponente mit einer Kohlendioxid bildenden Komponente und ggf. weiteren Hilfsstoffen und Additiven gemischt. Vorzugsweise werden die oben genannte bevorzugten Säurekomponenten und Kohlendioxid bildenden Komponenten eingesetzt. In einer für den Fachmann bekannten Weise werden die Mengen an sauren Bestandteilen mit den Mengen an CO₂-bildenden Bestandteilen so festgelegt, daß die resultierende Brausezubereitung bei Auflösung in Wasser den gewünschten pH-Wert ergibt.

Die Reaktivität des Brausekörpers kann der Fachmann in bekannter Weise dadurch steuern, daß er gut wasserlösliche und weniger gut wasserlösliche saure Bestandteile für das Brausegranulat auswählt. Dies kann z.B. in bewährter Weise durch ein Gemisch von gut wasserlöslicher Zitronensäure und relativ schlecht löslichem Mononatriumcitrat erfolgen. Die Lösegeschwindigkeit der sauren Komponente und damit die Reaktivität des Brausegranulats kann auch durch die Kristallgröße z.B. der Zitronensäure gesteuert werden. Selbstverständlich wird die Zerfallszeit der Brausetablette nicht nur über die Reaktivität des Brausekörpers, sondern auch über die eingesetzte Menge geregelt.

Üblicherweise werden bei der Herstellung der Brausegranulate die oben genannten weiteren Hilfsstoffe eingesetzt. Damit nicht bereits bei der Granulation eine Reaktion zwischen der sauren Komponente und der CO₂-bildenden Komponente eintritt, müssen die Bestandteile des Brausegranulats entweder mit organischen Lösungsmitteln wie z.B. Äthanol und Isopropanol granuliert werden, oder es dürfen nur ganz geringe Mengen an Wasser zugesetzt werden, die möglichst rasch z.B. durch das Anlegen von Vakuum aus dem befeuchteten Brausekörper-Granulat wieder zu entfernen sind.

Wie bereits beschrieben, werden die CO₂-bildende Komponenten, insbesondere Natriumhydrogencarbonat, mit den sauren Bestandteilen, vorzugsweise im wesentlichen Zitronensäure und Mononatriumcitrat, zu einem Brausekörpergranulat umgesetzt. Es können jedoch auch saure und basische Bestandteile einzeln der Endmischung zugesetzt werden. Beispielsweise können zur Korrektur des pH-Werts der entstehenden Brauselösung oder zur Beschleunigung der Brausereaktion der Endmischung noch geringe Mengen einer Säure und/oder einer CO₂-bildenden Komponente zugesetzt werden. Die genannten Mengen an CO₂-bildende Komponenten und sauren Bestandteilen liegen zu 60 bis 100 Gew.-%, bevorzugt 75 bis 90 Gew.-% als Brausegranulat vor, der Rest wird ggf. der Endmischung zugesetzt.

Im folgenden wird die Erfindung anhand von Ausführungsbeispielen weiter erläutert.

### Ausführungsbeispiele

### Beispiel 1: Herstellung eines ibuprofenhaltigen Granulats mit K₂CO₃/Na₂CO₃ (1:0,042) unter Zugabe von Isopropanol

In einem 1400 Liter Vakuumgranulator wurden 360 kg Ibuprofen mit einer mittleren Korngröße von 0,075 mm, 270 kg Kaliumcarbonat mit einer mittleren Korngröße von 0,09 mm, 9 kg Natriumcarbonat mit einer mittleren Korngröße von 0,05 mm eingefüllt und ca. 5 Minuten gemischt. Über zwei 0,5 mm Einstoffdüsen wurden 7,5 kg Isopropanol innerhalb von 10 Minuten eingesprüht. Das Produkt wurde kontinuierlich über weitere 30 Minuten gerührt, wobei sich die Produkt-Temperatur von 22 auf maximal 53 °C erhöhte. Das Produkt blieb unter Rühren stets pulverförmig und ging innerhalb von 40 Minuten in ein feines, fließfähiges Granulat mit einer mittleren Korngröße von ca. 0,08 mm über. Eine repräsentative Probe des Granulats löste sich in Wasser innerhalb von Sekunden klar auf. Die Umwandlung des schwerlöslichen Ibuprofens in ein hoch wasserlösliches Ibuprofen-Kalium-Salzgemisch war beendet. Legte man jetzt für 5 Minuten Vakuum an, reduzierte sich die Produkt-Temperatur auf 35-40 °C und der Trocknungsverlust war < 0,5 % (im wesentlichen Isopropanol). Dieses Granulat kann bereits für die Herstellung von Brausetabletten eingesetzt werden. Verlängerte man die Trockenzeit auf 15 Minuten, erhielt man einen Trocknungsverlust von weniger als 0,2 %.

Zur Herstellung eines groberen Ibuprofen-Granulats, das sich z.B. zum Einsatz in 400 oder 600 mg Brausetabletten oder zur Abfüllung in Saches und Stickpacks eignet, wurde das Material durch Sprühen von weiteren Isopropanol granuliert. Dabei reichte eine eingesprühte Menge von 10-25 kg aus. Nach kurzer Antrocknung mittels Vakuum wurde das entstandene grobere Granulat gesiebt und danach auf den gewünschten Trocknungsverlust getrocknet. Die Trocknungszeit betrug für einen Trocknungsverlust von ca. 0,5 % ca. 25 Minuten, für einen Trocknungsverlust < 0,2 % ca. 60 Minuten. Die mittlere Korngröße des Granulates betrug 0,25 mm mit einer Korngrößenverteilung zwischen 0,1 und 1,25 mm.

Auf ein mol Ibuprofen wurden 1,17 mol der basischen Komponenten Kaliumcarbonat und Natriumcarbonat eingesetzt, das Verhältnis der kaliumhaltigen zu dem natriumhaltigen basischen Hilfsstoffe beträgt 1 : 0,042.

Der Wirkstoffgehalt des Granulats betrug 100,5 % (bezogen auf den theoretischen Sollwert von 56,3 %).

### Beispiel 2: Bestimmung der Lösungsgeschwindigkeit des Granulats aus Beispiel 1

In einer Dissolution-Apparatur gemäß der Europäischen Pharmacopoe (Methode 2) wurde in den Gefäßen 500 ml demineralisiertes Wasser von 20 °C vorgelegt. Die Geschwindigkeit des Paddle war auf 100 Umdrehungen pro Minute eingestellt. Die Auflösezeit für die unter (a) hergestellten Granulate betrug:
mittlere Korngröße 0,08 mm 8 Sekunden
mittlere Korngröße 0,25 mm 19 Sekunden.

Es wurde jeweils eine repräsentative Probe von 1 g eingesetzt.

### Beispiel 3: Herstellung eines wirkstoffhaltigen Granulats mit K₂CO₃/Na₂CO₃ (1:0,96) unter Zugabe von Isopropanol

Exakt nach Beispiel 1 wurden in den Vakuumgranulator 412 kg Ibuprofen, 155 kg Kaliumcarbonat, 115 kg Natriumcarbonat, 32 kg Mannit eingefüllt und für 5 Minuten gemischt. Unter Rühren wurden 8 kg Isopropanol eingesprüht und 55 Minuten gerührt. Die Masse erreichte nach 50 Minuten eine Temperatur von 54 °C. Nach ca. 30 Minuten Rührzeit wurde für wenige Minuten die gerührte Masse leicht klebrig und ging nach weiteren 10 Minuten in ein feines Granulat über. Es entstand eine geringfügige Menge von groberen Granulatagglomeraten in der Größenordnung bis 2,5 cm, so daß die Masse nach kurzer Antrocknungszeit von 3 Minuten durch ein Sieb mit der Maschenweite 2,5 mm und danach von 1,0 mm gesiebt wurde. Das gesiebte Granulat wurde in den Vakuumgranulator zurückgeführt und innerhalb von 45 Minuten auf einen Trocknungsverlust von < 0,3 % getrocknet.

Auf 1 Mol Ibuprofen wurden 1,1 Mol basische Komponenten eingesetzt. Das Verhältnis der kaliumhaltigen zu den natriumhaltigen basischen Hilfsstoffen beträgt 1 : 0,96. Die gemäß Beispiel 2 bestimmte Auflösezeit von 1 g des Ibuprofengranulats beträgt 28 Sekunden (mittlere Korngröße 0,4 mm). Lösungsmittelmenge (bezogen auf Gesamtmasse) 1,1 %.

Beim Beispiel 3 lag der Gehalt an Ibuprofen bei 101,2 % (bezogen auf den theoretischen Soll-Wert von 57,7 %. Dieser Gehalt beweist, daß die Reaktion praktisch ausschließlich über die Carbonate allein abgelaufen ist. Die während der Reaktion gebildeten Kaliumhydrogencarbonat und Natriumhydrogencarbonat haben praktisch nicht mit Ibuprofen unter Bildung von CO₂ und Wasser reagiert.

### Beispiel 4: Herstellung eines wirkstoffhaltigen Granulats ohne Zugabe von organischem Lösungsmittel in einem Rollerkompaktor

200 kg (970 mol) Ibuprofen, 140 kg (1013 mol) Kaliumcarbonat und 24 kg Trinatriumphosphat (wasserfrei, 145,5 mol) wurden gemischt und gleichmäßig in den Trichter eines Rollerkompaktors gefüllt. Durch die Wirkung des Drukkes (ca. 10 KN) und die dabei entstehende Wärme, einschließlich der Reaktionswärme während der Kompaktierung wurde eine Masse gebildet, die kurz nach der Abkühlung durch Siebe mit Maschenweiten von 2,5 und 1,25 mm gesiebt wurden konnte und vollständig in Wasser löslich war. Die beiden Kompaktor-Rollen waren wassergekühlt.

Auf 1 Mol Ibuprofen wurden 1,19 Mol alkalische Hilfsstoffe eingesetzt. Das Verhältnis zwischen den alkalischen Kalium- und Natriumsalzen betragt 1 : 0,15. Das Granulat löste sich in der unter Beispiel 2 beschriebenen Apparatur unter den gleichen Versuchsbedingungen in 22 Sekunden auf. Die mittlere Korngröße betrug 0,6 mm. Das IbuprofenGranulat ist bestens zur Herstellung von Brausetabletten oder zur Abfüllung in Saches ggf. zusammen mit weiteren Hilfsstoffen und wie Aromen und Süßstoffen geeignet.

### Beispiel 5: Herstellung eines wirkstoffhaltigen Granulats ohne Zugabe von organischem Lösungsmittel im Vakuumgranulator

In einem heiz- und kühlbaren 1400 Liter-Vakuumgranulator wurden nacheinander eingefüllt:
412 kg Ibuprofen (2000 mol), 276,0 kg Kaliumcarbonat (2000 mol), 14,6 kg Lysin (100 mol). Der Heizmantel des Vakuumgranulators wurde auf 50 °C eingestellt. Während einer Rührzeit von 90 Minuten stieg die Produkt-temperatur bis auf 68 °C an. Das Produkt war zu diesem Zeitpunkt völlig wasserlöslich. Es wurde für 10 Minuten Vakuum angelegt und dann auf eine Produkt-Temperatur von 35 °C gekühlt. Es war ein feines Granulat mit einigen größeren Agglomeraten entstanden. Deshalb wurde das Material durch ein Sieb mit einer Maschenweite von 1,25 mm gesiebt.

Das Verhältnis Ibuprofen/wasserlösliche alkalische Hilfsstoffe betrug 1 : 1,05. Der Gehalt an Ibuprofen betrug 102,7 % (bezogen auf 58,7 % Ibuprofen in der Ausgangsmischung). Der Anstieg des Gehaltes weist darauf hin, daß während der bei einer etwas höheren Temperatur durchgeführten Umsetzung kleine Mengen des gebildeten Kaliumhydrogencarbonates unter Bildung von CO₂ und Wasser mit Ibuprofen reagiert haben.

Ein repräsentatives 1 g Muster löste sich entsprechend der im Beispiel 2 beschriebenen Methode in 500 ml Wasser von 20 °C innerhalb von 12 Sekunden auf (mittlere Partikelgröße 0,2 mm). Das Granulat ist bestens geeignet für die Herstellung von Brausetabletten oder zur Herstellung von Granulaten, die vor der Einnahme in Wasser gelöst werden.

Der Reaktionsmischung wurde kein Wasser oder Lösungsmittel zugesetzt.

### Beispiel 6: Herstellung eines wirkstoffhaltigen Granulats mit K₂CO₃/Na₂CO₃ (1:0,092) im Extruder

In das 1. Segment eines Doppelschneckenextruders wurden pro Stunde 41,2 kg Ibuprofen und eine Mischung aus 30,0 kg Kaliumcarbonat und 2,1 kg Natriumcarbonat dosiert. Das 2. - 7. Segment des Doppelschneckenextruders waren auf eine Reaktionstemperatur von 100 °C eingestellt. Im 6. Segment des Extruders wurden 20 kg Maltit pro Stunde gravimetrisch zudosiert. Das 8.-10. Segment des Extruders war zur Kühlung des solubilisierten Produktes auf 30 °C eingestellt. Die mittlere Verweilzeit des zudosierten Ibuprofens und der wasserlöslichen basischen Hilfsstoffe im Extruder betrug ca. 2 Minuten. Am Extruderende wurde ein noch schwach klebriges Granulat ausgestoßen, das nach wenigen Minuten durch ein Sieb mit einer Maschenweite von 2,0 mm gesiebt werden konnte.

Die extrudierte Masse war hervorragend klar wasserlöslich. Das Verhältnis von Ibuprofen zu alkalische Hilfsstoffe betrug 1 : 1,19, das Verhältnis zwischen Kaliumsalz und Natriumsalz 1 : 0,092. Der Wirkstoffgehalt der solubilisierten Masse betrug (bezogen auf den Ausgangsgehalt an Ibuprofen von 44,16 %) 103,1 %. Durch die schärferen Reaktionsbedingungen, die aufgrund der kurzen Verweilzeit im Extruder notwendig sind, war die Reaktion weniger eindeutig und ein Teil des gebildeten Natriumhydrogencarbonats und ggf. kleine Mengen an gebildetem Kaliumhydrogencarbonat setzten sich in CO₂ und Wasser um.

Die rasterelektronenmikroskopische Untersuchung des Granulates zeigte, daß das Granulat eine bienenwabenähnliche Struktur hatte, mit Hohlräumen von ca. 30 µm Durchmesser. Hierbei handelte es sich offensichtlich um eingeschlossene CO₂- Mikrobläschen. Dies ist auch der Grund dafür, daß dieses extrudierte Granulat hervorragend zur Herstellung von Saches oder Stickpacks geeignet ist. Schüttete man dieses Granulat in Wasser, so begann es nach wenigen Sekunden unter Auflösung an die Wasseroberfläche zu schwimmen, offensichtlich aufgrund des eingeschlossenen Kohlendioxids.

Das extrudierte Granulat mit einer mittleren Korngröße von 0,8 mm löste sich unter den in Beispiel 2 beschriebenen Bedingungen innerhalb von 26 Sekunden auf.

### Beispiel 7: Herstellung eines Brausegranulats

In einen Vakuumgranulator wurden
- 105,8 kg: Zitronensäure
- 133,4 kg: Mononatriumcitrat
- 501,4 kg: Natriumhydrogencarbonat
- 18,4 kg: Saccharin-Natrium
- 4,6 kg: Saccharosepalmitat
- 13,8 kg: Hydroxypropylmethylcellulose
gefüllt und für 10 Minuten gemischt. In die Mischung wurden mit einer Einstoffdüse 4,6 kg Ethanol gesprüht und für weitere 15 Minuten gerührt. Es entstand ein feinstrukturiertes Granulat. Der Wassermantel des Vakuumgranulators wurde auf 60 °C und das Vakuum auf ca. 100 mbar eingestellt. Nach einer Trocknungszeit von ca. 75 Minuten wurde das Produkt auf ca. 30 - 35 °C abgekühlt und das Brausegranulat entladen. Das Granulat wurde durch ein Sieb mit einer Maschenweite von 1,25 mm gesiebt und in dicht schließenden Behältern gelagert.

### Beispiel 8: Herstellung von Brausetabletten

In einem geeigneten Mischbehälter wurden 710 kg Ibuprofengranulat gemäß Beispiel 1, 1690 kg Brausegranulat gemäß Beispiel 7, 250 kg Natriumhydrogencarbonat, 100 kg Zitronensäure, 10 kg Menthol-Aroma (163592 Symrise), 40 kg Grapefruit-Aroma (3018177 Symrise) eingefüllt und für 15 Minuten gemischt.

Aus der Mischung wurden auf bekannte technische Weise die folgenden Brausetabletten hergestellt:

| **Tablette** | **Ibuprofengehalt** | **Durchmesser** | **Tablettengewicht** |
|---|---|---|---|
| | **[mg]** | **[mm]** | **[mg]** |
| **1** | 200 | 18 | 1400 |
| **2** | 400 | 25 | 2800 |
| **3** | 600 | 25 | 4200 |

Alle Tabletten lösten sich innerhalb von 2 - 3,5 Minuten klar auf. Die Härte der Tabletten betrug 50 N für die 200 mg Brausetablette und ca. 60 -90 N für die 400 und 600 mg Brausetabletten. Nach der genannten Zerfallszeit war praktisch kein Bodensatz sichtbar. Der Zerfall der Tablette ging mit einer sofortigen und vollständigen Auflösung der in der Tablette befindlichen Granulate einher. Es bildeten sich praktisch keine groben Wirkstoffpartikel, die nach dem Zerfall der Brausetablette noch sichtbar wären. Besonders bemerkenswert ist der gegenüber Konkurrenztabletten vom Markt hervorragende Geschmack der 200 mg Brausetablette, die klare Auflösung ohne sichtbare, schwimmende Wirkstoffpartikel.

### Beispiel 9: Herstellung von Trinkgranulat

400 kg Ibuprofengranulat gemäß Beispiel 6 wurden mit 80 kg Brausegranulat gemäß Beispiel 7 gemischt. Der Mischung wurden 10 kg Mentholaroma (163592 Symrise) und 40 kg Grapefruit-Aroma (301877 Symrise) zugefügt. Die Mischung wurde in sogenannte Stickpacks mit nachfolgenden Gewichten abgefüllt:
- 200 mg Dosierung:: 601 mg
- 400 mg Dosierung:: 1202 mg
- 600 mg Dosierung:: 1803 mg
- 800 mg Dosierung:: 2404 mg

Schüttete man den Inhalt eines Sticks in ein Glas mit 200 ml Wasser, 20 °C, so lösten sich alle Dosierungen selbständig ohne Rühren innerhalb von 30 - 90 Sekunden auf und waren danach trinkfertig.

## Patentansprüche

1. Ibuprofen-Brausezubereitung enthaltend
(a) ein wirkstoffhaltiges Granulat, das Ibuprofen und einen basischen Hilfsstoff enthält, und
(b) ein Brausegranulat, das eine Säurekomponente und eine Kohlendioxid bildende Komponente enthält,
**dadurch gekennzeichnet, daß** das wirkstoffhaltige Granulat das Ibuprofen in Form eines wasserlöslichen Salzes enthält.

2. Brausezubereitung nach Anspruch 1, in der das wirkstoffhaltige Granulat das Kaliumsalz von Ibuprofen enthält.

3. Brausezubereitung nach Anspruch 2, in der das wirkstoffhaltige Granulat zusätzlich das Natriumsalz von Ibuprofen enthält.

4. Brausezubereitung nach Anspruch 3, die das Kaliumsalz und das Natriumsalz von Ibuprofen in einem molaren Verhältnis von Kalium- zu Natriumsalz von 1:0 bis 1:1 enthält.

5. Brausezubereitung nach einem der vorhergehenden Ansprüche, in der die basische Komponente des wirkstoffhaltigen Granulats NaHCO₃, KHCO₃, Na₂CO₃, K₂CO₃, Na₂HPO₄, K₂HPO₄, Dinatriumcitrat, Dikaliumcitrat, die protonierte Form von Lysin, Arginin, Kaliumglycinat, Natriumglycinat, Meglumin oder eine Mischung davon enthält.

6. Brausezubereitung nach Anspruch 5, in der die basische Komponente des wirkstoffhaltigen Granulats KHCO₃ oder eine Mischung von KHCO₃ und NaHCO₃ enthält.

7. Brausezubereitung nach einem der vorhergehenden Ansprüche, in der das Verhältnis von Ibuprofensalz zu basischem Hilfsstoff in dem wirkstoffhaltigen Granulat im Bereich von 0,8 bis 2 mol basischem Hilfsstoffs pro mol Ibuprofensalz liegt.

8. Brausezubereitung nach einem der vorhergehenden Ansprüche, in der das Brausegranulat als Säurekomponente Zitronensäure, Weinsäure, Apfelsäure, Ascorbinsäure, ein saures Salz dieser Säuren, ein saures anorganisches Salz, Glutaminsäure, Adipinsäure, Glutaminsäure Hydrochlorid, Betainhydrochlorid oder eine Mischung davon enthält.

9. Brausezubereitung nach einem der vorhergehenden Ansprüche, in der das Brausegranulat als Kohlendioxid bildende Komponente ein Alkali- oder Erdalkalihydrogencarbonat oder -carbonat enthält.

10. Brausezubereitung nach einem der vorherigen Ansprüche in Form einer Dosierungseinheit.

11. Brausezubereitung nach Anspruch 10 in Form einer Brausetablette oder von in Sachets oder Stickpacks abgefülltem Granulat.

12. Brausezubereitung nach Anspruch 10 oder 11, die 200 bis 800 mg Ibuprofen (gemessen als freie Säure) pro Dosierungseinheit enthält.

13. Brausezubereitung nach einem der Ansprüche 10 bis 12 in Form einer Brausetablette, wobei eine Brausetablette ein Gesamtgewicht von 1000 bis 1500 mg pro 200 mg Ibuprofen (gemessen als freie Säure) aufweist.

14. Brausezubereitung nach einem der Ansprüche 10 bis 12 in Form eines Trinkgranulats, wobei eine Dosierungseinheit des Trinkgranulats ein Gesamtgewicht von 500 bis 950 mg pro 200 mg Ibuprofen (gemessen als freie Säure) aufweist.

15. Brausezubereitung nach einem der vorhergehenden Ansprüche, die bezogen auf einen Gewichtsteil Ibuprofen 2 bis 4 Gewichtsteile Alkalihydrogencarbonat enthält.

16. Brausezubereitung nach einem der vorhergehenden Ansprüche, bei der das wirkstoffhaltige Granulat (a) gemäß einem Verfahren der nachfolgenden Ansprüche herstellbar ist.

17. Verfahren zur Herstellung einer Brausezubereitung gemäß einem der vorhergehenden Ansprüche, bei dem man das wirkstoffhaltige Granulat herstellt, indem man Ibuprofen in Form seiner freien Säure mit einem oder mehreren basischen Hilfsstoffen ohne Zugabe von Wasser intensiv mischt.

18. Verfahren nach Anspruch 17, bei dem man das Ibuprofen und den basischen Hilfsstoff unter Zugabe eines organischen Lösungsmittel miteinander mischt.

19. Verfahren nach Anspruch 18, bei dem man als organisches Lösungsmittel Isopropanol, Äthanol, Aceton, oder eine Mischungen davon zugibt.

20. Verfahren nach Anspruch 18 oder 19, bei dem man das organische Lösungsmittel in einer Menge von 0,5 bis 10 Gew.-% bezogen auf das Gesamtgesicht der Reaktionsmischung zugibt.

21. Verfahren nach einem der Ansprüche 17 bis 20, bei dem man die Mischung von Ibuprofen und Hilfsstoffen auf eine Temperatur von 50 bis 60 °C erwärmt.

22. Verfahren nach einem der Ansprüche 17 bis 21, bei dem man als basischen Hilfsstoff eine wasserlösliche basische Substanz verwendet, die in 0,1 molarer wäßriger Lösung einen pH-Wert > 11,0 hat.

23. Verfahren nach Anspruch 22, bei dem man eine basische Substanz verwendet, die aus Trikaliumphosphat, Trinatriumphosphat, Trikaliumcitrat, Trinatriumcitrat sowie den entsprechenden Natriumsalzen, dem Kalium- und Natriumsalz des Glycins, Lysin, Arginin physiologisch unbedenklichen organischen Aminen, Meglumin, Alkalicarbonaten und Mischungen dieser Stoffe ausgewählt ist.

24. Verfahren nach Anspruch 23, bei dem man als basischen Hilfsstoff Kaliumcarbonat, Natriumcarbonat oder eine Mischung davon verwendet.

25. Verfahren nach einem der Ansprüche 17 bis 24, bei dem man als basischen Hilfsstoff eine Mischung von Kalium- und Natriumverbindungen in einem molaren Verhältnis (K : Na) von 1:0 bis 1:1 einsetzt.

26. Verfahren nach einem der Ansprüche 17 bis 25, bei dem man pro mol Ibuprofen 0,8 bis 2 mol basischen Hilfsstoff verwendet.

27. Verfahren nach einem der Ansprüche 17 bis 26 bei dem man das Mischen in einem Vakuumgranulator, Kompaktor, Wirbelschichtreaktor oder Extruder durchführt.

28. Verfahren nach einem der vorhergehenden Ansprüche, bei dem man das Granulat nach seiner Herstellung auf einen Wassergehalt von weniger als 0,5 Gew.-% trocknet.

29. Verfahren nach Anspruch 28, bei dem man das Granulat auf einen Wassergehalt von weniger als 0,25 Gew.-% trocknet.

30. Verfahren nach einem der Ansprüche 17 bis 29, bei dem man das wirkstoffhaltige Granulat anschließend mit einem Brausegranulat mischt.

31. Verfahren nach Anspruch 30, bei dem man die Mischung aus wirkstoffhaltigem Granulat und Brausegranulat in Sachets oder Stickpacks verpackt oder zu Tabletten verpreßt.
